Europäisches Patentamt

European Patent Office

Office européen des brevets

(19)

(11) EP 0 770 425 A1

(12) EUROPEAN PATENT APPLICATION

(43) Date of publication:
02.05.1997 Bulletin 1997/18

(51) Int Cl.6: **B01J 23/44**, B01J 23/96,
B01J 38/60

(21) Application number: 96306327.6

(22) Date of filing: 30.08.1996

(84) Designated Contracting States:
**BE DE FR GB IT NL**

(30) Priority: **27.10.1995 GB 9521997**

(71) Applicant: **BP Chemicals Limited
London EC2M 7BA (GB)**

(72) Inventor: **Froom, Simon Frederick Thomas,
BP Chemicals Ltd.
Humberside HU12 8DS (GB)**

(74) Representative:
**Krishnan, Suryanarayana Kalyana et al
BP INTERNATIONAL LIMITED
Patents & Agreements Division
Chertsey Road
Sunbury-on-Thames Middlesex TW16 7LN (GB)**

(54) **Activation and regeneration of hydrogenation catalysts**

(57) This invention relates to a process for activating or regenerating a hydrogenation catalyst comprising palladium on carbon for the hydrogenolysis of a trialkoxy propane of the formula

$$RO.CH_2.CH_2.CH(OR)_2 \qquad (II)$$

wherein R is an alkyl group having 1 to 4 carbon atoms. The process comprises subjecting the catalyst to a treatment in a medium comprising an acid having a pKa value of less than 6 and then washing the treated catalyst until it has a pH value greater than 2 when 1 part of the dry catalyst is suspended in 40 parts of de-ionised water.

EP 0 770 425 A1

**Description**

The present invention relates to a process for the activation of a fresh and/or regeneration of a used hydrogenation catalyst comprising palladium on carbon and more particularly, to catalysts used for the hydrogenation of trialkoxy propane to the corresponding 1,3-dialkoxypropane.

It is reported in "Acrolein", C W Smith, Editor, John Wiley & Sons (1962) at page 114 that the diethyl acetal of ethoxypropionaldehyde undergoes hydrogenolysis in the presence of Raney nickel to form the diethyl ether of 1,3-propanediol. The following equation is given:

$$C_2H_5OCH_2CH_2CH(OC_2H_5)_2 \rightarrow C_2H_5OCH_2CH_2CH_2OC_2H_5 + C_2H_5OH$$

US Patent 2,425,042 discloses the catalyzed reaction

$$(R_1O)_2C(R)CH(OR_1)_2 + 2H_2 \rightarrow R_1OCH(R)CH_2OR_1 + 2R_1OH$$

wherein R is hydrogen or methyl and $R_1$ is an alkyl, aralkyl, alkoxyalkyl, alkoxyalkenyloxyalkyl or alkoxy-polyalkenyloxy-alkyl radical. The reference indicates that the preferred catalyst is Raney nickel, although other hydrogenation catalysts such as copper-chromium oxide, platinum and palladium black can be used.

US Patent 2,590598 discloses the catalytic hydrogenation reaction

$$R"CH(OR')[CH_2CH(OR)]_nCH_2CH(OR)_2 \rightarrow R"CH(OR')[CH_2CH(OR)]_nCH_2CH_2OR + ROH$$

wherein each R represents the same or different hydrocarbon radicals chosen from the group consisting of alkyl, aryl and aralkyl radicals, and n is a whole number including 0 and generally less than 10. The reference indicates that Raney nickel is the preferred catalyst but "other metal hydrogenation catalyst (ie, platinum or palladium) or copper, chromium etc." can be used. A particular aspect of this disclosure is that there is no disclosure of the hydrogenation of a propane ether nor the use of a supported catalyst.

US Patent 4,479,017 discloses the catalyzed hydrogenation reaction

$$[R(YO)_n]_2C(R^1)(R^2) \rightarrow R(YO)_nCH(R^1)(R^2)$$

wherein R represents a hydrogen atom or a lower alkoxy group, Y represents an alkylene group having 2 to 12 carbon atoms, n is a positive number of 1 to 6, the two groups $R(YO)_n$ may, together with the carbon atom to which they are bonded, represent a 1,3-dioxolane ring, and $R^1$ and $R^2$, independently from each other, represent a hydrogen atom or an alkyl group having 1 to 6 carbon atoms, provided that at least one of $R^1$ and $R^2$ represents a hydrogen atom. The catalyst is a "palladium catalyst supported on a carbon carrier in the absence of an acidic substance added". Moreover, this reference states that, for their hydrogenation reaction, a palladium on carbon catalyst when used in the absence of acid cocatalyst is superior to the same catalyst when used in the presence of an acid cocatalyst. Furthermore, this reference states that, for their hydrogenation reaction, a palladium-on-alumina catalyst in the absence of an acid substance added cannot achieve the excellent improvement shown by palladium on carbon catalyst of that invention. This reference does not disclose the hydrogenation of ether acetals of the 1,1,3-trialkoxy alkane type nor the effect of the additional ether group in such compounds on the hydrogenation reaction.

Our prior published International Application No. WO/9501949 describes a process for making a 1,3-diether compound represented by the formula:

$$RO.CH_2.CH_2.CH_2.OR \qquad\qquad (I)$$

wherein R is an alkyl group having 1 to 4 carbon atoms, the process comprising hydrogenating an ether acetal of the formula

$$RO.CH_2.CH_2.CH(OR)_2 \qquad\qquad (II)$$

wherein R has the same meaning as in formula (I) above, in the presence of a catalyst composition comprising at least one catalytic metal selected from the group consisting of Pd, Ni, Co, Pt, Rh and Ru and a support material, said support material being one or more selected from the group consisting of silica, alumina, silica-alumina, aluminosilicates and carbon.

Thus, in this type of reaction, it is well known eg from US-A-4479017 referred to above that the Pd/C hydrogenation catalyst used should be free from acid since the presence of significant amounts of acid may destabilise and cause decomposition of the triethoxypropane (II).

One of the problems with the Pd/C hydrogenation catalyst in this type of reaction is that it is deactivated during the reaction. Due to the expensive nature of the catalyst, it is essential for an economically viable process that the deactivated catalyst is reactivated and reused in the process.

Surprisingly, it has now been found that whilst the presence of acid is detrimental to the hydrogenation reaction, the Pd/C hydrogenation catalyst

(i) when fresh can be activated and/or
(ii) when deactivated can be regenerated to its initial activity

if said catalyst is treated with an acid and then the acid removed therefrom prior to (re)use in the hydrogenation process.

Accordingly, the present invention is a process for activating or regenerating a hydrogenation catalyst comprising palladium on carbon for the hydrogenolysis of a trialkoxy propane of formula

$$RO.CH_2.CH_2.CH(OR)_2 \qquad\qquad (II)$$

said process comprising subjecting the catalyst to a treatment in a medium comprising an acid having a pKa value of less than 6 and then washing the treated catalyst until it has a pH >2 when 1 part of the dry catalyst is suspended in 40 parts of de-ionised water.

In the trialkoxy propane of formula (II) which is to be hydrogenated, R preferably represents an ethyl group. Such compounds and their methods of preparation are well known in the art. For instance, see our prior published International Application No. WO/9501949. The matter disclosed in this Application is incorporated herein by reference.

Thus, trialkoxy alkanes of formula (II) can be produced by reacting an unsaturated aldehyde, eg acrolein, with an alcohol, R.OH, with at least one acidic reagent for an effective period of time to form the desired trialkoxy propane. The molar ratio of the aldehyde to alcohol is preferably about 1:1 to 1:20, more preferably about 1:3 to about 1:10.

The reaction to produce the trialkoxy propane from the unsaturated aldehyde can be conducted in a batch, continuous or semi-continuous mode in a fixed bed or a slurry reactor.

The reaction to form the trialkoxy propane is suitably conducted at a temperature in the range of about 0°C to about 120°C, preferably from about 20°C to about 80°C. The reaction pressure is suitably in the range of about 0 kPa to about 700 kPa, preferably from about 0 kPa to about 150 kPa. The average residence time of the aldehyde with the acidic reagent is preferably from about 5 minutes to about 20 hours.

This reaction produces a mixture of products principally containing the unreacted aldehyde and alcohol, the corresponding alkoxyaldehyde and the unsaturated acetal, the desired trialkoxy propane and water. The desired trialkoxy propane is recovered by separation from the other organic components, any residual acid and most of the water present in the reaction products. The trialkoxy propane so recovered is suitably free of or rendered free of any acid and is converted to the diether by catalytic hydrogenation.

The hydrogenation reaction is suitably carried out at hydrogen pressures of about 650 kPa to about 35,000 kPa, preferably from about 1,500 kPa to about 20,000 kPa, more preferably from about 3,000 kPa to about 17,500 kPa eg from about 4,500 kPa to about 7,500 kPa. The mole ratio of the trialkoxy propane (II) to hydrogen can be from about 1:1 to about 1:100, and is preferably from about 1:2 to about 1:50.

The hydrogenation catalyst comprises palladium metal on a carbon support. The carbon, where used, can be one of the many forms of carbon eg graphite or activated carbon. The catalytic metal may be deposited or impregnated on the support using conventional mixing or precipitation techniques. The catalyst suitably has a palladium content of about 0.05% w/w to about 20% w/w. The weight ranges quoted above are based solely on the weight of the catalytic metal and the support and does not take into account any water or moisture content associated with either component. Examples of commercially available catalysts that are useful include *inter alia* Escat®10 (ex Engelhard Industries) which contains 5% w/w of palladium on activated carbon powder and has a surface area of 850 $m^2/g$. One sample had a moisture content of about 52.5% w/w although this parameter is not critical for the success of the invention.

The hydrogenation reaction may optionally be carried out in the presence of a solvent. Examples of such solvents include *inter alia* alcohols, ethers and esters. Polar solvents such as alcohols are preferred although in this case the alcohol used should be such that the oxyalkyl group of the alcohol solvent corresponds to the oxyalkyl group in the desired 1,3-diethyl ether (I). A sufficient amount of solvent can be used to dilute the trialkoxy propane (II) to the desired concentration to facilitate handling and/or to maintain the reaction mass in solution.

The hydrogenation reaction is suitably carried out at a temperature in the range from about 50°C to about 250°C, preferably from about 100°C to about 225°C and more preferably from about 125°C to about 225°C, eg from about 150°C to about 210°C.

The hydrogenation of the trialkoxy propane with hydrogen in the presence of a catalyst as described above can be carried out in a slurry reactor, a fixed bed reactor, a spouted bed reactor or any other suitable reactor configuration such as eg a moving bed reactor. The reactants may be in a gaseous phase and/or a liquid phase. The reaction can be carried out in a continuous, semi-continuous or a batch-type mode.

The average residence time of the acetal reactant (II) in contact with the catalyst during the formation of the corresponding 1,3-diether compound (I) is suitably from about 5 minutes to about 30 hours, preferably from about 15 minutes to about 10 hours.

In the various embodiments of the process of the present invention to produce the 1,3-diether compound of formula (I), one or more of the following compounds may be generated as by-products:

a)   R.OH
b)   3-alkoxy propanol-1
c)   1-propanol and
d)   alkyl propyl ether

Of these, (a) is always a generated as a product during the hydrogenation of the acetal. However, the present process has the significant advantage that it is particularly selective towards the formation of the desired 1,3-diether (I) relative to the formation of the by-products (b)-(d) above. Thus, in one embodiment, the selectivity of the inventive process towards the formation of (I) relative to the formation of the combined amounts of(b)-(d) is at least about 60 mole % and is preferably at least about 90 mole %.

One of the problems of the above hydrogenation process is the rapidity with which the hydrogenation catalyst is deactivated. This deactivation is signified by the reduction of the rate of hydrogen uptake during the hydrogenation reaction. The deactivated catalyst may be reactivated according to the present invention by bringing the deactivated catalyst into contact with an acidic reagent. Such a treatment can also be applied to a fresh catalyst in order to maximise its activity.

The acidic reagent used for treating the fresh or used catalyst has a pKa value <6, preferably <5. Usually such acids can be characterised by dissolving them in water to form at least a 0.01 moles/litre solution and is identified as an acid by Methyl Red indicator. The acidic reagents that may be used include one or more of mineral acids such as hydrochloric acid, sulphuric acid, fluoro-sulphonic acid or phosphoric acid; one or more C1-C7 (i) mono- or dicarboxylic acids or (ii) halogenated fatty acids; and alkyl and aryl sulphonic acids such as eg methane sulphonic acid and para-toluene sulphonic acid.

The activation procedure comprises contacting an amount of the catalyst to be activated with one or more acids ( as defined above) either as such or as a solution thereof in a neutral solvent such as eg water. The choice of acid(s) and its concentration will depend upon the degree of activation required and the nature of the palladium/carbon catalyst. The weight ratio of the catalyst to acid is suitably in the range from 1:100 to 10:1.

The duration of contact between the acid and the catalyst to be activated will depend upon the degree of activation required and the nature of the acid used. Where a catalyst is activated using a solution of an acid, after due contact, any surplus acidic liquid is suitably removed by known methods such as filtration or centrifugation and decantation. It is preferable to remove as much of the acid, which may be adsorbed thereon, from the reacted/activated catalyst by efficiently washing the acid-treated catalyst with sufficient neutral solvent such as eg water, methanol or ethanol. This solvent washing step may, if necessary, be repeated several times until the activated catalyst is substantially free of acid contamination as hereinbefore defined.

Thus, the acid treated catalyst after washing with a neutral solvent has a pH value >2, preferably >3, when 1 part of the dry catalyst is suspended in 40 parts of de-ionised water. This can be ascertained by thoroughly mixing 0.5 g ofthe dry treated catalyst with 20 ml of distilled water and measuring the pH of the resultant solution/slurry. If the measured pH is >2, preferably >3 then the catalyst is ready for re-use, otherwise further washing with the neutral solvent may be required.

As described above, the re-activated catalyst shows a substantial improvement in the rate of hydrogen uptake during the hydrogenation of the trialkoxy propane. The present invention is further illustrated with reference to the following Examples.

**EXAMPLES:**

**EXAMPLE 1**

**A. Hydrogenation of 1,1,3-Triethoxy propane**

A 150 gallon stirred stainless steel autoclave was purged with nitrogen (3 x 103 kPa (15 psi)) and then charged with 542 kg of 1,1,3-triethoxypropane and, following a further nitrogen purge (2 x 103 kPa (15 psi)), further charged with dry Escat® 10 (0.8 kg, 5% Pd on carbon ex Engelhard Co). After purging the autoclave with hydrogen (3 x 206 kPa (30 psi)), the autoclave was pressurised to 2413 kPa (350 psi) with hydrogen and heating commenced. When the temperature of the autoclave contents reached 150°C, hydrogen pressure was increased to 4481 kPa (650 psi) and maintained at this level until the temperature reached 170-180°C. These conditions were then maintained for 10 hours. Thereafter, the autoclave and its contents were cooled and the contents discharged via a Calmic filter. GC analysis of the products in the filtrate indicated that the selectivity to 1,3-diethoxy propane was 97% and about 2.2 % of the 1,1,3-triethoxy propane reactant remained unconverted. The filter cake, containing the catalyst, was wetted with water in order to facilitate its safe handling.

Two further batches of 1,1,3-triethoxy propane were hydrogenated in a similar manner and similar results were obtained. The used, wet catalyst from each of these three runs was recovered and found to be deactivated by the following experiments:

**Experiment 1:**

A portion of the wet catalyst recovered from the reaction described in Example 1(A) above, was washed thoroughly with absolute ethanol, under an nitrogen (inert) atmosphere, in order to displace the water and then dried in a stream of nitrogen. 0.188 g of this recovered catalyst after washing and drying as described above was charged together with 1,1,3-triethoxy propane (300 ml) into a stirred zirconium autoclave (500 ml capacity) equipped with a baffle cage and ballast vessel. An initial pressure of 200 kPa (2 barg) hydrogen was applied and the reactor contents heated to 160°C whereupon, the hydrogen pressure was increased to 4800 kPa (48 barg) and maintained under these conditions for 1240 minutes. The time taken for the ballast vessel pressure to decline by 600 kPa (6 barg) (representing hydrogen uptake corresponding to a little under half total theoretical uptake) was 168 minutes. Analysis of the reaction product by GC gave a calculated triethoxy propane conversion of 97% and a selectivity to 1,3-diethoxy propane of 96%.

**Experiment 2:**

The process of Experiment 1 was repeated except that fresh, unused Escat® 10 catalyst was employed. The total reaction time was 335 minutes and analysis of the product led to a calculated triethoxy propane conversion of 99% and a selectivity to diethoxy propane of 96%. Significantly, the time taken for the ballast vessel pressure to drop by 600 kPa (6 barg) (representing hydrogen uptake) was only 58 minutes providing clear evidence that the fresh catalyst had higher catalytic activity.

**B. Reactivation of the Used/Deactivated Catalyst:**

**Experiment 3:**

A 2 g portion of the recovered catalyst from Example 1(A) above was washed by thoroughly mixing the catalyst portion with glacial acetic acid (3 x 30 ml) and then absolute ethanol (3 x 30 ml) on a glass frit of low porosity. Following each occasion that a slurry was formed, surplus liquid was drawn off to leave a cake ready for the next portion of liquid, or, finally, drying under a stream of nitrogen.

Experiment 1 was repeated but using 0.187 g of the catalyst portion as washed above. In this instance the time taken for the ballast vessel pressure to drop by 600 kPa (6 barg) ( corresponding to hydrogen uptake) took only 104 minutes. The total reaction time allowed was 342 minutes and analysis of the product indicated a triethoxy propane conversion of 83% and a selectivity to diethoxy propane of 96%.

**EXAMPLE 2:**

**Experiment 4:**

A 2 g portion of the recovered catalyst from Example 1(A) above was washed by thoroughly mixing the catalyst portion with 1 molar sulphuric acid (3 x 30 ml) and then de-ionised water (3 x 30 ml) and finally with absolute ethanol (3 x 30 ml) on a glass frit of low porosity. Following each occasion that a slurry was formed, surplus liquid was drawn off to leave a cake ready for the next portion of liquid, or, finally, drying under a stream of nitrogen.

Experiment 1 was repeated but using 0.187 g of the catalyst portion as washed above. In this instance the time taken for the ballast vessel pressure to drop by 600 kPa (6 barg) (corresponding to hydrogen uptake) took only 47 minutes. The total reaction time allowed was 368 minutes and analysis of the product indicated a complete (100%) conversion of triethoxy propane and a selectivity to diethoxy propane of 97%.

These results show that the rate of hydrogen uptake of the re-activated catalyst was far superior to that prior to reactivation and that there is no significant loss of conversion or selectivity of the re-activated catalyst.

| Ex. No. | By- Products (% w/w) | | Conv. % of TREP | Selec. % to DE3 | Press. (kPa) | Temp. (°C) | Catalyst | Loading (w/v %) | Time (minutes) | |
|---|---|---|---|---|---|---|---|---|---|---|
| | EtOPr | EP | | | | | | | Reaction | Δ600 kPa |
| 1* | 0.2 | 2.3 | 97 | 96 | 4800 | 160 | Escat®10-Recycle | 0.06 | 1242 | 168 |
| 2* | 0.4 | 1.2 | 99 | 97 | 4800 | 160 | Escat®10 | 0.06 | 335 | 58 |
| 3 | 0.3 | 0.6 | 83 | 96 | 4800 | 160 | Escat®10-Recycle (Acetic acid) | 0.06 | 342 | 104 |
| 4 | 0.7 | 0.8 | 100 | 97 | 4800 | 160 | Escat®10-Recycle (Sulphuric Acid) | 0.06 | 368 | 47 |

EtOPr - 3-Ethoxy propanol
EP - 3-Ethoxy propanol
TREP - 1,1,3-Triethoxy propane
DE3 - 1,3-Diethoxy propane
* - Comparative Experiments not according to the invention.

EP 0 770 425 A1

**Claims**

1. A process for activating or regenerating a hydrogenation catalyst comprising palladium on carbon for the hydrogenolysis of a trialkoxy propane of the formula

$$RO.CH_2.CH_2.CH(OR)_2 \hspace{4cm} (II)$$

wherein R is an alkyl group having 1 to 4 carbon atoms, said process comprising subjecting the catalyst to a treatment in a medium comprising an acid having a pKa value of less than 6 and then washing the treated catalyst until it has a pH value greater than 2 when 1 part of the dry catalyst is suspended in 40 parts of de-ionised water.

2. A process according to Claim 1 wherein R in formula (II) represents an ethyl group.

3. A process according to Claim 1 or 2 wherein the hydrogenolysis reaction is conducted at hydrogen pressures in the range from 650 kPa to 35,000 kPa and at a temperature in the range from 50°C to 250°C.

4. A process according to any one of the preceding Claims wherein the mole ratio of the trialkoxy propane (II) to hydrogen is in the range from 1:1 to 1:100.

5. A process according to any one of the preceding Claims wherein the hydrogenation catalyst comprises palladium metal on a carbon support and has a palladium content in the range from 0.05% w/w to 20% w/w.

6. A process according to any one of the preceding Claims wherein the carbon support for the catalyst is activated carbon powder which has an average surface area of about 850 $m^2$/g.

7. A process according to any one of the preceding Claims wherein the supported catalyst whether fresh or deactivated is (re)activated by bringing the supported catalyst into contact with an acidic reagent which has a pKa value <5 using a weight ratio of the catalyst to acid in the range from 1:100 to 10:1.

8. A process according to any one of the preceding Claims wherein the acidic reagent comprises one or more of mineral acids selected from hydrochloric acid, sulphuric acid, fluoro-sulphonic acid or phosphoric acid; one or more C1-C7 (i) mono- or di-carboxylic acids or (ii) halogenated fatty acids; and alkyl and aryl sulphonic acids selected from methane sulphonic acid and para-toluene sulphonic acid.

9. A process according to any one of the preceding Claims wherein the catalyst is activated with one or more acids either as such or as a solution thereof in a neutral solvent.

10. A process according to any one of the preceding Claims wherein the acid treated catalyst is washed with a neutral solvent until it has a pH value greater than 2 when 1 part of the dry catalyst is suspended in 40 parts of de-ionised water.

European Patent
Office

**EUROPEAN SEARCH REPORT**

Application Number

EP 96 30 6327

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | US-A-4 717 696 (ARENA BLAISE J) 5 January 1988 *all document* | | B01J23/44 B01J23/96 B01J38/60 |
| A | FR-A-1 479 886 (STANDARD OIL COMPANY) 28 March 1967 *all document* | | |
| A | GB-A-1 150 422 (STANDARD OIL COMPANY) 30 April 1969 *all document* | | |

TECHNICAL FIELDS
SEARCHED      (Int.Cl.6)

B01J

The present search report has been drawn up for all claims

| Place of search | Date of completion of the search | Examiner |
|---|---|---|
| THE HAGUE | 13 January 1997 | Faria, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document